# EUROPEAN PATENT APPLICATION

(11) **EP 4 592 291 A1**
(43) Date of publication of application: **30.07.2025**
(21) Application number: 23876214.0
(22) Date of filing: 21.06.2023
(51) Int. Cl.: C07D 487/04, C09K 11/06, G01N 21/64

(54) **USE OF PEMETREXED NEAR-INFRARED FLUORESCENT MOLECULE TARGETING ALPHA-TYPE FOLATE RECEPTOR AS FLUORESCENT TRACER**

(30) Priority: 13.10.2022 CN 202211251552
(71) Applicant: Nanjing Nuoyuan Medical Devices Co., Ltd, Nanjing, Jiangsu 210000 (CN)
(72) Inventor: CAI, Huiming, Nanjing, Jiangsu 210000 (CN); WANG, Yiqing, Nanjing, Jiangsu 210000 (CN); LI, Yunlong, Nanjing, Jiangsu 210000 (CN)
(74) Representative: Cleanthous, Marinos
(86) International application number: PCT/CN2023/101595
(87) International publication number: WO 2024/078004

(57) **Abstract**

Disclosed is use of a pemetrexed near-infrared fluorescent molecule targeting a folate receptor α as a fluorescent tracer. The structural formula of the pemetrexed near-infrared fluorescent molecule is 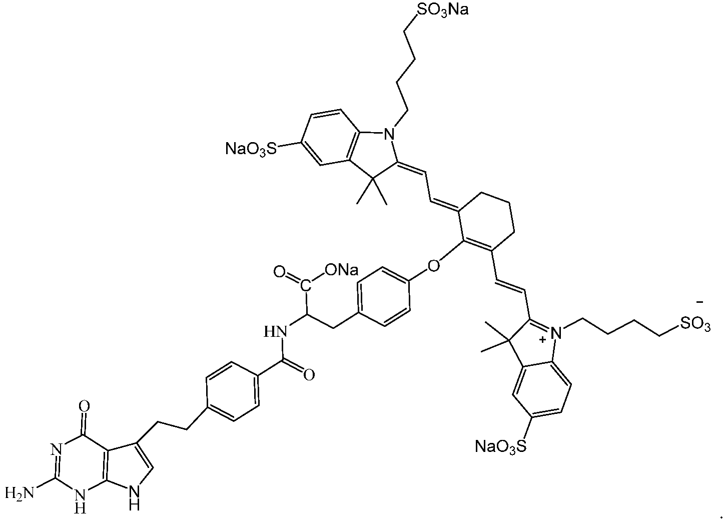 The active targeting near-infrared molecule has relatively low affinity to a folate receptor β while retaining high affinity to the folate receptor α, so as to effectively differentiate a tumor from inflammation. Meanwhile, the active targeting near-infrared molecule has the advantages of good aqueous solubility, high fluorescent quantum yield and the like, and possesses a huge development potential in the fields of near-infrared fluorescence image tumor surgery navigation imaging and medical cell markers.

## Description

### TECHNICAL FIELD

The present disclosure relates to use of a novel pemetrexed near-infrared fluorescent tracer targeting a folate receptor α for differentiating a non-small cell lung cancer with high folate receptor α expression from inflammation, and relates to the fields of near-infrared surgery navigation fluorescent molecule and cell marker imaging and the like.

### BACKGROUND

In recent years, near-infrared (NIR) imaging has been widely applied to clinical tumor imaging. Currently, indocyanine green (ICG) is the only fluorescent agent which is approved by the China Food and Drug Administration (CFDA) and the US Food and Drug Administration (FDA) and used for human medical imaging and clinical diagnosis in clinical practice. ICG is a hydrophilic tricarbocyanine dye with a molecular weight of 776. In an aqueous solution, ICG has a maximum absorption peak at 785 nm and a maximum emission peak at 810-820 nm. It is reported that ICG can provide minimally invasive imaging of tumors and metastatic lymph nodes, including real-time imaging during the surgery. It is generally believed that ICG binds to albumin in serum to form 4-6 nm nano particles, and enriches in tumors through its enhanced permeability and retention (EPR) effector. However, ICG is not preferentially accumulated in tumor tissues because it is not a tracer specific to a receptor. It has found that ICG can be accumulated not only in tumors but also in other high-permeability tissues, such as inflammatory tissues. Studies show that ICG also exhibits fluorescence in inflammatory tissues near tumors, which has become a main restriction for ICG application. To improve this situation, there is a need to design a near-infrared fluorescent tracer with active targeting.

The targeted delivery of a fluorescent probe to tumor cancer cells by utilizing the specific binding characteristics of folic acid and a folate receptor has received widespread attentions from scholars both domestically and internationally. However, there are four folate receptor subtypes: FRα, β, γ and δ, two of which, i.e., FRα and β, are highly expressed in cancer tissues, wherein FRα is mainly over-expressed in malignant tumors of epithelial tissues, such as ovarian cancer, lung cancer and uterine cancer; and FRβ is up-regulated in placenta, monocytes and macrophages, and the above-mentioned two subtypes are simultaneously expressed in the same organism. Since patients suffering from malignant tumors may also be afflicted by inflammatory diseases, and FRβ is highly expressed due to accumulation and activation of macrophages in inflammatory areas. Folic acid and folic acid conjugates have similar affinity to the two subtypes of folate receptors, so that they cannot selectively recognize tumor cells or inflammatory areas. Therefore, modification of folic acid and folate conjugates to improve their specificity for tumor targeting has important practical application significance.

Studies have found that there is great difference between pemetrexed's affinities to FRα and FRβ. By utilizing this characteristic, for tumor cancer cells with high folate receptor α expression, an FRα efficiently mediated targeting marker system is obtained by selecting a pemetrexed derivative as a targeting gene. Compared with the traditional targeting marker system, the above obtained targeting marker system is more efficiently transported to tumor cells expressing FRα while reducing the targeting interference of inflammatory cells expressing FRβ subtype on the targeting system, which has important significance for quick and efficient detection of tumor cells.

### SUMMARY

The objective of the present disclosure is to provide use of a pemetrexed near-infrared fluorescent molecule targeting a folate receptor α as a fluorescent tracer to achieve quick and efficient detection of tumor cells.

In order to achieve the above-mentioned objective, the present disclosure adopts the following technical solution:

Provided is use of a pemetrexed near-infrared fluorescent molecule targeting a folate receptor α as a fluorescent tracer, wherein the structural formula of the pemetrexed near-infrared fluorescent molecule is

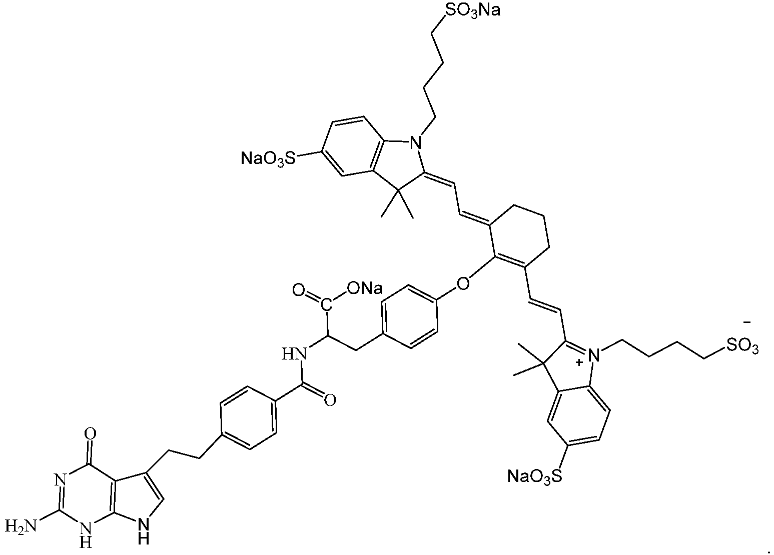

The fluorescent tracer effectively differentiates a tumor from an inflammation tissue by utilizing different specificities to different folate receptors.

The fluorescent tracer is capable of differentiating a non-small cell lung cancer with high folate receptor α expression from inflammation.

The fluorescent tracer has relatively low affinity to a folate receptor β while retaining high affinity to the folate receptor α.

The present disclosure has the following beneficial effects: an active targeting near-infrared fluorescent small molecule with pemetrexed disodium and derivatives thereof as active targeting groups is prepared by utilizing an organic total synthesis method, and is innovatively applied to differentiation of tumor and inflammatory tissue imaging. Such the active targeting near-infrared fluorescent molecule has relatively low affinity to the folate receptor β while retaining high affinity to the folate receptor α, so as to differentiate a tumor from inflammation. Meanwhile, the active targeting near-infrared fluorescent molecule has the advantages of good aqueous solubility, high fluorescent quantum yield and the like, and possesses a huge development potential in the fields of near-infrared fluorescence image tumor surgery navigation imaging and medical cell markers.

### BRIEF DESCRIPTION OF THE DRAWINGS

FIG. 1 is a synthesis flowchart of a pemetrexed near-infrared fluorescent molecule;

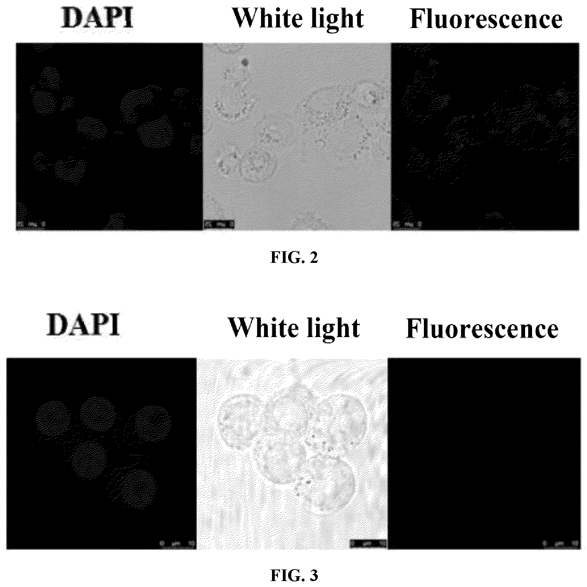

FIG. 2 is a cell graph showing high expression of a folate receptor α targeted by a pemetrexed near-infrared fluorescent molecule;
FIG. 3 is a cell graph showing a high expression of a folate receptor β targeted by a pemetrexed near-infrared fluorescent molecule;

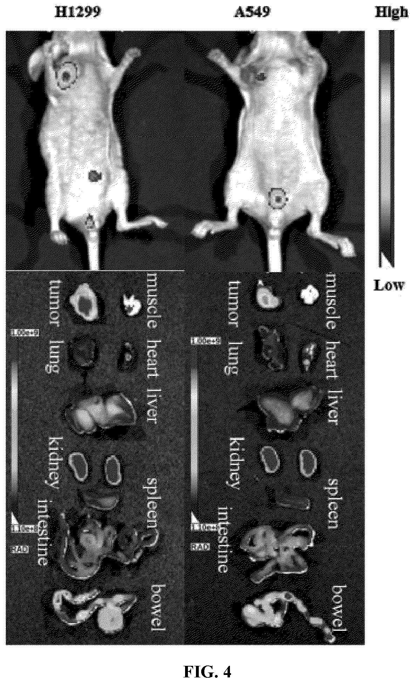

FIG. 4 is an in vivo imaging graph of a tumor with high expression and low expression of a pemetrexed near-infrared fluorescent molecule folate receptor α;
FIG. 5 is a fluorescent image of differentiating a non-small cell lung cancer with high expression of a pemetrexed near-infrared fluorescent molecule folate receptor α from inflammation;
FIG. 6 shows HE, FRα and FRβ immunohistochemistry and fluorescence images of tumor slices; and
FIG. 7 shows HE, FRα and FRβ immunohistochemistry and fluorescence images of inflammatory tissue slices.

### DETAILED DESCRIPTION OF THE EMBODIMENTS

The present disclosure will be further explained below in conjunction with drawings.

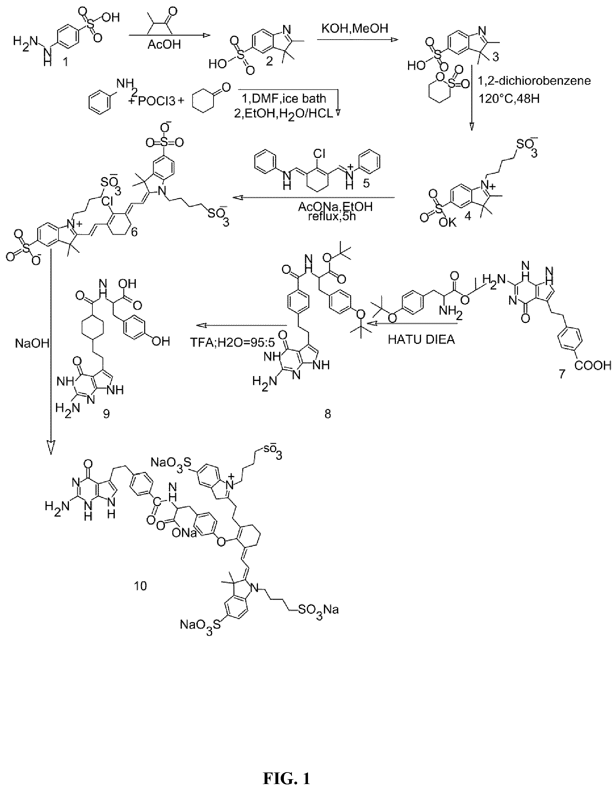

As shown in FIG. 1, a pemetrexed near-infrared fluorescent molecule was prepared, comprising the following steps:

### 1. Synthesis of a chlorinated ICG derivative parent

(1) 4-hydrazine benzenesulfonic acid (1.6 g, 31.9 mmol), 3-methyl-2-butane (2.10 ml, 90 mmol) and glacial acetic acid (50 ml) were mixed and heated to 120°C, and the obtained mixture was placed for 18 h under a nitrogen atmosphere. After the mixture was precipitated in ethyl acetate, a crude product was filtered and collected in a form of pink solid. The obtained product (6.5 g, 25.4 mmol) was dissolved into methanol (50 mL). Under the moderate condition, the dissolved solution was dropwise added into a solution of potassium (1.7 g, 30 mmol) and isopropanol (20ml). The obtained crude mixture was filtered and washed to obtain a brown solid (97%). 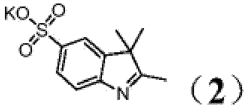
(2) A compound 2 (2.3 g, 8.3 mmol) and 1,4-butanesulfonic acid lactone were added into a toluene solution under the nitrogen atmosphere and heated for 48 h at 110°C. The above mixture was cooled to room temperature, and a solvent was dissolved out. Methanol (10 ml) was added into the crude mixture and stirred for 30 min, and then the crude mixture was filtered, collected, and dissolved in a mixture of water (10 ml) and methanol (50 ml) with a ratio of 2: 1 (v/v). The mixed solution was slowly added into acetonitrile (160 ml) by using a dropping funnel. A precipitate was filtered and collected as a pink solid.
   (40%) 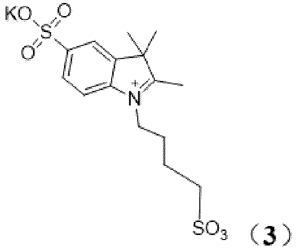
(3) A compound 3 (1.5 g, 2.79 mmol), a Vilsmeier-Haack reagent (0.5 g, 1.39 mmol) and absolute sodium acetate (0.342 g, 4.17 mmol) were subjected to reflux heating for 6 h under the nitrogen atmosphere in 20 mL of absolute ethanol. The reaction mixture was cooled to room temperature, followed by filtering and washing with ethanol and methanol, and then the obtained product was collected as a brown green solid. (90%)

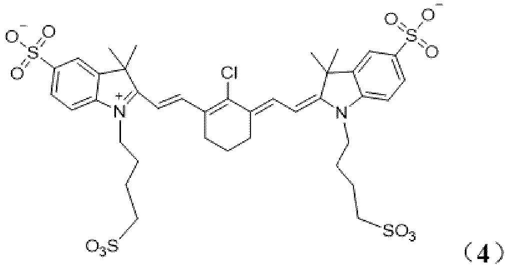

2. Synthesis of a pemetrexed target drug: pemetrexed hydrolyzed acid (1.05 g, 3.52 mmol) was dissolved into dimethyl formamide (DMF) under the stirring, 2-(7-azabenzotriazol-1-yl)-N,N,N',N'-tetramethyluronium hexafluorophosphate (HATU) (2.007 g, 5.28 mmol), O-tert-butyl-L-tyrosine tert butyl ester hydrochloride (1.161 g, 3.52 mmol) and diisopropylethylamine (DIEA) (1.364 g, 10.56 mmol) were successively added into a flask, stirred until being completely dissolved and reacted for 30 min at room temperature under the protection of nitrogen, then the solution after reaction was dropwise added into 0.1 N of aq.HCl (1.0 L, 0.14 M) to generate a faint yellow precipitate, the obtained precipitate was filtered at reduced pressure and dried in vacuum to obtain 52.04 g of solid compound, with a yield of 95%.

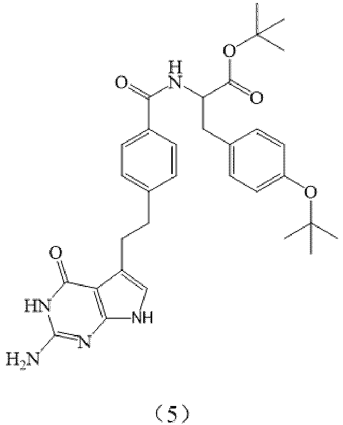

A compound 5 (2.04 g, 3.34 mmol) was put into a round-bottom flask, TFA: H₂O (95: 5 10 mL) was added, the above materials were stirred for 2 h, the solution after stirring was then added into methyl tert-butyl ether, and the obtained mixture was precipitated, filtered and dried in vacuum to obtain 1.507 g of compound, with a yield of 98%.

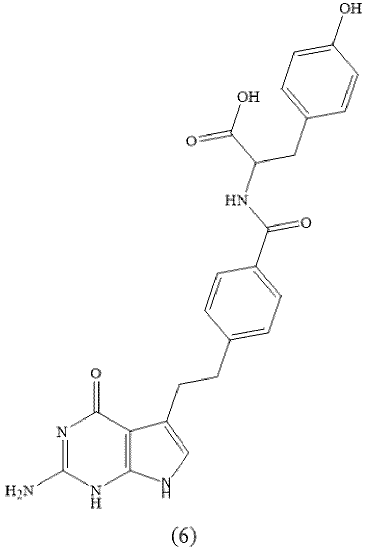

3. Synthesis of a pemetrexed near-infrared fluorescent molecule 7: Pemetrexed-Tyr (1.507 g, 3.276 mmol) trianion solution with pH 11 was dropwise added into (18 mL) aqueous solution of S0456 (2.909 g, 3.276 mmol) at 23°C. The reaction mixture was heated to 90°C and then stirred for 45 min at 90°C, and then the formation of the compound 7 was monitored through thin layer chromatography (TLC). After the formation of the product was completed, the reaction mixture was cooled to room temperature and served as a stabilizing flow to be transferred to acetone (0.5 L) being stirred through a casing pipe to obtain a green precipitate. The precipitate was filtered in vacuum via a sintering funnel on an air extractor and then washed with acetone (3×500 mL). The green powdered solid was dried in high vacuum for 12 h and then measured to obtain a compound 7 (4.34 g).

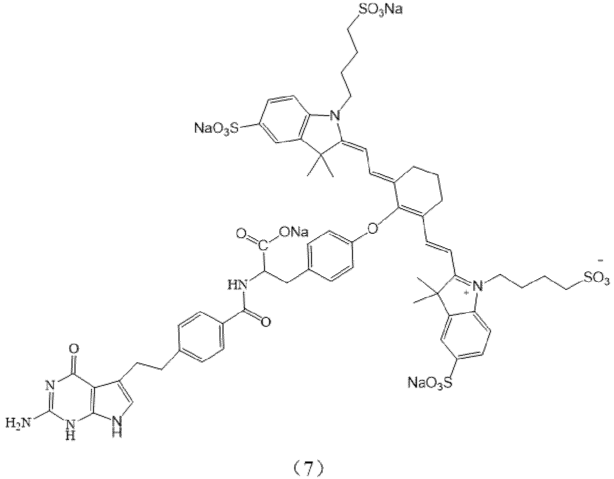

4. In order to verify the effect of the present disclosure, the following verification experiment was conducted:
(1) Use of a pemetrexed near-infrared fluorescent molecule in the aspect of differentiating a tumor with high folate receptor α expression from inflammation, that is, specific recognition of the pemetrexed near-infrared fluorescent molecule on a cell line with high folate receptor α expression: a cell line H1299 with high folate receptor α expression, a cell line A549 with low folate receptor α expression and a cell line RAW264.7 with high folate receptor β expression were selected. The cells were cultured in a Roswell Park Memorial Institute (RPPMI) culture medium containing 10% fetal bovine serum (FBS) and 1% diabody in moist air containing 5% CO₂ and 95% air at 37°C. The cells were incubated for 1 h in a confocal vessel by utilizing a 100 nM pemetrexed near-infrared fluorescent tracer. The cells after incubation were washed with PBS, stained with DPAI, fixed with paraformaldehyde and then photographed.

The pemetrexed near-infrared fluorescent tracer was seen in the entire cytoplasm of the H1299 cell, but was not observed in the folate receptor α negative A549 cell, proving that the pemetrexed near-infrared fluorescent tracer was internalized by the cells with high folate receptor α expression, and an argument that the pemetrexed near-infrared fluorescent tracer specifically targets the cells with high folate receptor α expression was supported. Fluorescence was not detected in RAW264.7, showing that the pemetrexed near-infrared fluorescent tracer did not target a folate receptor β.

(2) The pemetrexed near-infrared fluorescent molecule was used for specifically recognizing non-small lung cancer tissues with high folate receptor α expression: the pemetrexed near-infrared fluorescent tracer was administrated to H1299 and A549 tumor-bearing mice via tail vein injection at a dose of 10 nm. After 2 h, imaging effects were observed through an animal imaging instrument and tissue biological distribution of mice was analyzed. The pemetrexed near-infrared fluorescent molecule was capable of specifically recognizing H1299 tumor-bearing mice with high folate receptor α expression and a good tumor background-to-signal ratio was observed, and the targeting effect of the A549 tumor-bearing mice with low folate receptor α expression was not detected.

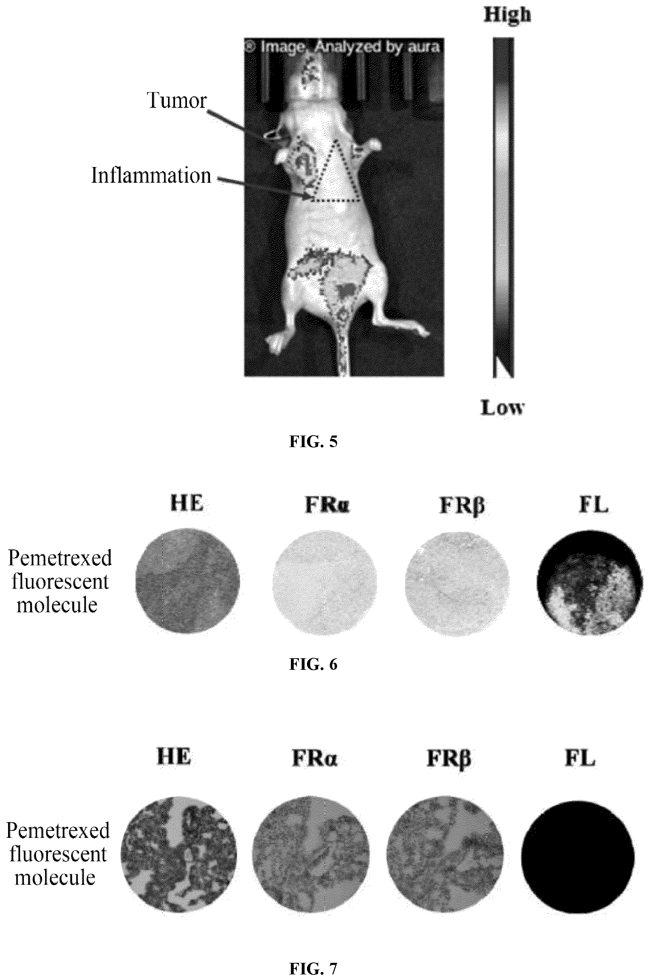

(3) The pemetrexed near-infrared fluorescent molecule was used for differentiating non-small cell lung cancer tissues with high folate receptor α expression from inflammation: an H1299 tumor-bearing mouse model was established, persulfate was injected in a peritoneal trigonum everyday for stimulation to generate peritonitis, as shown in FIG. 5, the trigonum of the site where inflammation was located was near the tumor tissue. The establishment of the inflammation model was verified by utilizing immunohistochemistry slices. It can be seen from the immunohistochemistry slices of tumor and inflammatory slices that a brown part represents positive receptor expression, the folate receptor α is highly expressed in the tumor, the folate receptor β is highly expressed in the inflammation, as shown in FIG. 6, the non-small cell lung cancer slice with high folate receptor α expression has relatively high intensity. FIG. 7 shows that the inflammatory tissue slice with high folate receptor β expression almost has no fluorescence. By utilizing different specificities to different folate receptor subtypes, the tumors are effectively differentiated from the inflammatory tissues.

The above-mentioned descriptions are only preferred embodiments of the present disclosure. It should be noted that for persons of ordinary skill in the art, several improvements and modifications can also be made without departing from the principle of the present disclosure, and these improvements and modifications should also be considered as the scope of protection of the present disclosure.

## Claims

1. Use of a pemetrexed near-infrared fluorescent molecule targeting a folate receptor α as a fluorescent tracer, wherein the structural formula of the pemetrexed near-infrared fluorescent molecule is 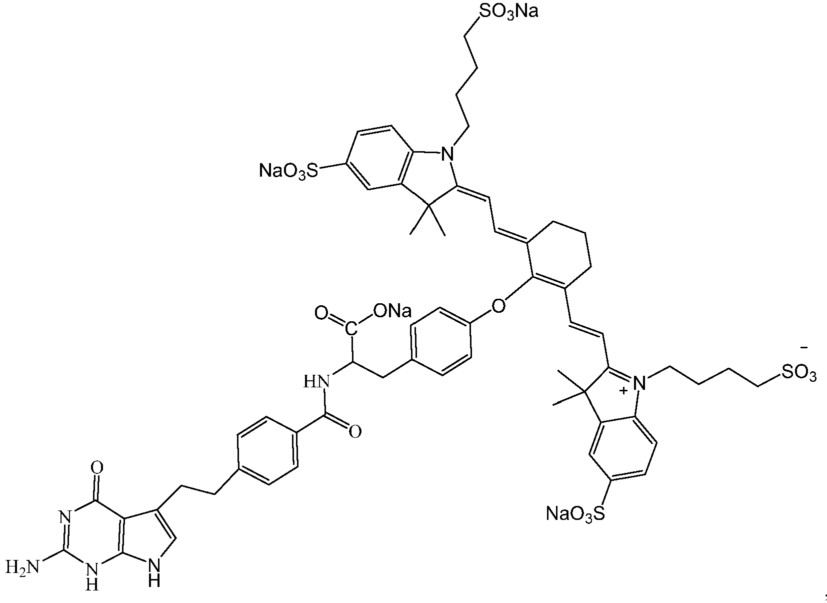 and
the fluorescent tracer effectively differentiates a tumor from an inflammation tissue by utilizing different specificities to different folate receptors.

2. The use according to claim 1, wherein the fluorescent tracer effectively differentiates a tumor from an inflammation tissue by utilizing different specificities to different folate receptors.

3. The use according to claim 2, wherein the fluorescent tracer is capable of differentiating a non-small cell lung cancer with high folate receptor α expression from inflammation.

4. The use according to claim 3, wherein the fluorescent tracer has relatively low affinity to a folate receptor β while retaining high affinity to the folate receptor α.
